# FASCICULE DE BREVET EUROPEEN

(11) **EP 3 630 736 B1**
(45) Date de publication et mention de la délivrance du brevet: **20.04.2022**
(21) Numéro de dépôt: 17740427.4
(22) Date de dépôt: 24.05.2017
(51) Int. Cl.: C07D 265/16

(54) **BENZOXAZINE HALOGÉNÉE UTILISABLE POUR LA SYNTHÈSE DE POLYBENZOXAZINE**
HALOGENIERTES BENZOXAZIN ZUR VERWENDUNG BEI DER SYNTHESE VON POLYBENZOXAZIN
HALOGENATED BENZOXAZINE FOR USE IN THE SYNTHESIS OF POLYBENZOXAZINE

(43) Date de publication de la demande: 08.04.2020
(73) Titulaire: Compagnie Générale des Etablissements Michelin, 63000 Clermont-Ferrand (FR)
(72) Inventeur: FEDURCO, Milan, 63040 Clermont-Ferrand Cedex 9 (FR); RIBEZZO, Marco, 63040 Clermont-Ferrand Cedex 9 (FR)
(74) Mandataire: Bocchi, Brigitte
(86) Numéro de dépôt international: PCT/FR2017/051293
(87) Numéro de publication internationale: WO 2018/215701

(56) Documents cités:
- WO-A1-2017/103376
- WO-A2-2010/002872
- CN-B- 103 058 948
- KISKAN, B., YAGGI, Y., ISHIDA, H.: "Synthesis, Characterization, and Properties of New Thermally Curable Polyetheresters Containing Benzoxazine Moieties in the Main Chain", JOURNAL OF POLYMER SCIENCE: PART A: POLYMER CHEMISTRY, vol. 46, 2008, pages 414-420, XP002773427,

## Description

### 1. DOMAINE DE L'INVENTION

La présente invention est relative aux monomères utilisables pour la synthèse de résines thermodurcissables, destinées notamment à des systèmes adhésifs permettant en particulier le collage de métal à du caoutchouc.

Elle est plus particulièrement relative aux composés benzoxazine aptes à la synthèse de polybenzoxazines utilisables notamment comme couches adhésives dans des composites métal / caoutchouc destinés à la fabrication d'articles en caoutchouc tels que des bandages, pneumatiques ou non pneumatiques, pour véhicules.

### 2. ETAT DE LA TECHNIQUE

Les composites métal / caoutchouc, en particulier pour bandages pour véhicules, sont bien connus. Ils sont le plus souvent constitués d'une matrice en caoutchouc généralement diénique, réticulable au soufre, comportant des éléments de renforcement (ou « renforts ») métalliques tels que des fils, films ou câbles en acier au carbone.

Soumis à des contraintes très importantes lors du roulage des bandages, notamment à des compressions, flexions ou variations de courbure répétées, ces composites doivent de manière connue satisfaire à un grand nombre de critères techniques, parfois contradictoires, tels qu'uniformité, flexibilité, endurance en flexion et en compression, résistance à la traction, à l'usure et à la corrosion, et maintenir ces performances à un niveau très élevé aussi longtemps que possible.

On comprend aisément que l'interphase adhésive entre caoutchouc et renforts joue un rôle prépondérant dans la pérennité de ces performances. Le procédé traditionnel pour relier les compositions de caoutchouc à de l'acier au carbone consiste à revêtir la surface de l'acier avec du laiton (alliage cuivre-zinc), la liaison entre l'acier et la matrice de caoutchouc étant assurée par sulfuration du laiton lors de la vulcanisation ou cuisson du caoutchouc. Pour améliorer l'adhésion, on utilise en outre généralement, dans ces compositions de caoutchouc, des sels organiques ou des complexes de métal tels que des sels de cobalt, en tant qu'additifs promoteurs d'adhésion.

Or, on sait que l'adhésion entre l'acier au carbone et la matrice de caoutchouc est susceptible de s'affaiblir au cours du temps, du fait de l'évolution progressive des sulfures formés sous l'effet des différentes sollicitations rencontrées, notamment mécaniques et/ou thermiques, le processus de dégradation ci-dessus pouvant être accéléré en présence d'humidité. D'autre part, l'utilisation de sels de cobalt rend les compositions de caoutchouc plus sensibles à l'oxydation et au vieillissement, et en augmente significativement le coût, sans compter qu'il est souhaitable de supprimer à terme l'emploi de tels sels de cobalt dans les compositions de caoutchouc, en raison de l'évolution récente de la réglementation européenne sur ce type de sels métalliques.

Pour toutes les raisons exposées ci-dessus, les fabricants de composites métal / caoutchouc, en particulier les manufacturiers de bandages pour véhicules, sont à la recherche de solutions adhésives nouvelles pour faire coller les renforts métalliques aux compositions de caoutchouc, tout en palliant, au moins en partie, les inconvénients précités.

C'est ainsi que les demandes WO 2014/063963, WO 2014/063968, WO 2014/173838, WO 2014/173839 récemment publiées, déposées par les Demanderesses, ont décrit des polymères nouveaux à unités urée, uréthane ou thiourée, ainsi que leurs monomères de départ, qui répondent aux objectifs ci-dessus. Utilisés notamment comme primaire d'adhésion sur métal dans des composites métal / caoutchouc, ces polymères permettent très avantageusement de coller le métal aux matrices de caoutchouc en utilisant ensuite de simples colles textiles telles que des colles « RFL » (résorcinol-formaldéhyde-latex) ou autres compositions adhésives équivalentes, ou encore directement (c'est-à-dire sans emploi de telles colles) à ces matrices de caoutchouc lorsque ces dernières contiennent par exemple des élastomères insaturés fonctionnalisés appropriés tels que des élastomères époxydés. Ainsi peuvent être supprimés notamment les sels de cobalt (ou autres sels métalliques) dans les compositions de caoutchouc destinées à être reliées à des renforts métalliques laitonnés.

Des polybenzoxazines sont décrites dans WO 2010/002872 et Kiskan, B. et al. Journal of Polymer Science: Part A: Polymer Science, vol. 46, 2008, pages 414-420.

Poursuivant leurs recherches, les Demanderesses ont trouvé un composé benzoxazine nouveau, utilisable comme monomère pour la synthèse d'une polybenzoxazine, du type thermodurcissable, qui à température ambiante présente les mêmes performances adhésives, vis-à-vis du métal et du caoutchouc, que les polymères précités mais qui présente une fois thermodurcie (réticulée) une stabilité thermique et chimique encore améliorée et dont la microstructure spécifique, en outre, permet très avantageusement d'ajuster la flexibilité de la molécule selon les applications particulières visées.

### 3. BREVE DESCRIPTION DE L'INVENTION

La présente invention concerne une benzoxazine halogénée répondant à la formule (le symbole « Hal » représentant un halogène) : 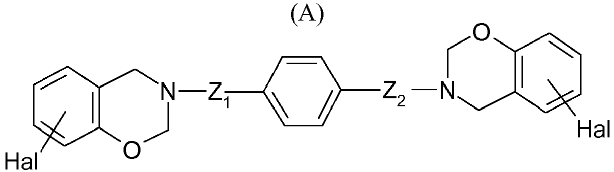 dans laquelle :
- chaque noyau benzénique des deux cycles oxazine est porteur d'au moins un halogène ;
- Z₁ et Z₂, identiques ou différents, représentent un groupement de liaison au moins divalent, aliphatique, comportant de 1 à 12 atomes de carbone

Grâce à cette benzoxazine spécifique, il est possible de préparer des polymères benzoxazine ou « polybenzoxazines » qui ont la capacité remarquable, à haute température, d'ouvrir leurs cycles oxazine et de conduire ainsi à une structure de résine polyphénolique thermodurcissable. Ceci leur confère, comparativement aux autres polymères connus décrits en introduction du présent mémoire, une meilleure stabilité thermique. Sa microstructure spécifique permet enfin, très avantageusement, d'ajuster la flexibilité des polybenzoxazines selon les applications particulières visées.

L'invention concerne également l'utilisation d'un composé conforme à l'invention pour la synthèse d'une polybenzoxazine, ainsi que toute polybenzoxazine issue d'au moins un composé benzoxazine selon l'invention.

L'invention concerne également tout procédé de synthèse d'une polybenzoxazine par polycondensation d'un composé selon l'invention, notamment avec, à titre de second monomère, un composé diol ou thiol aromatique.

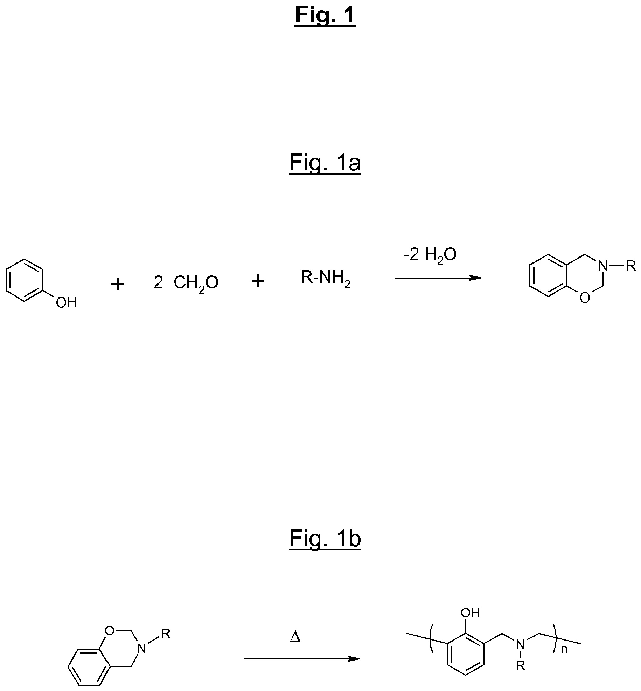

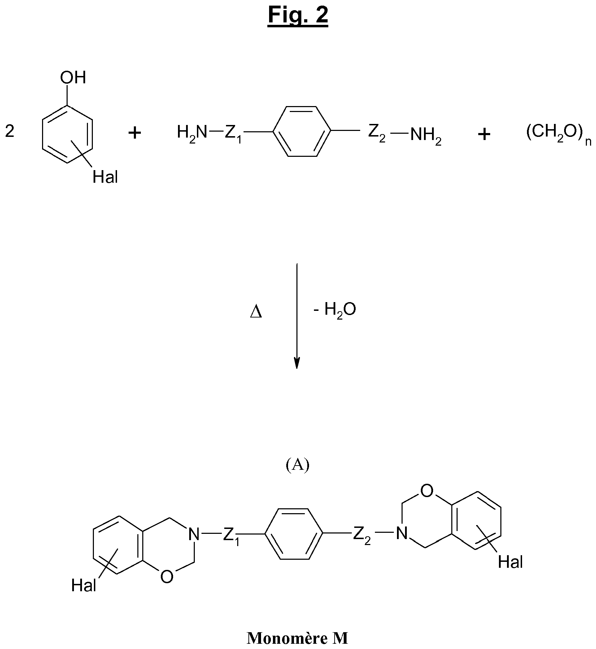

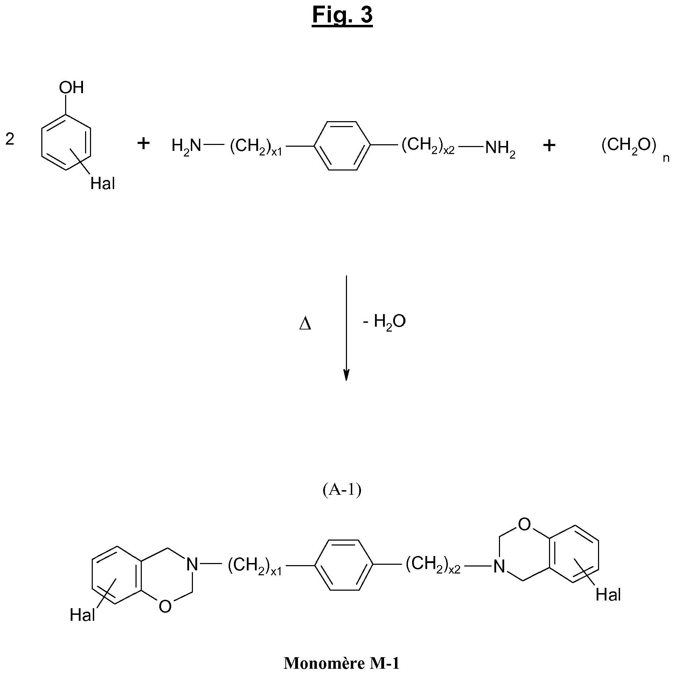

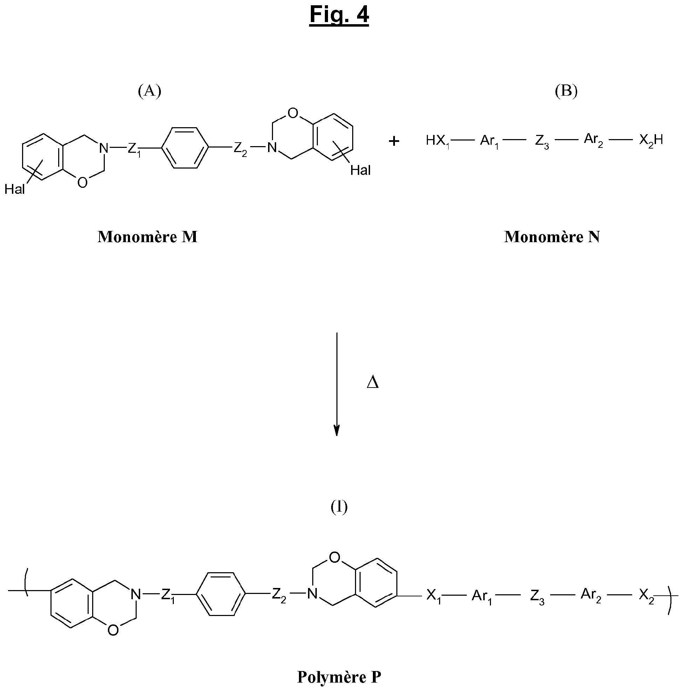

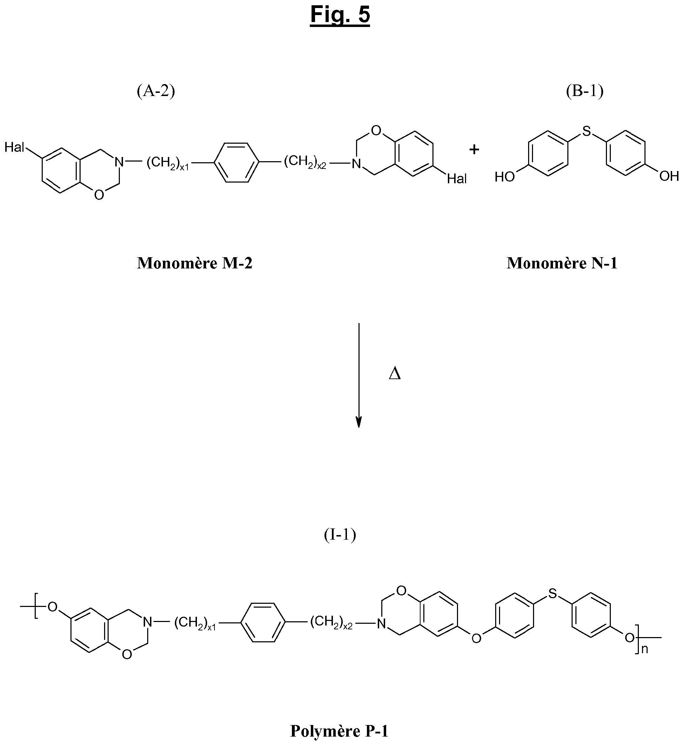

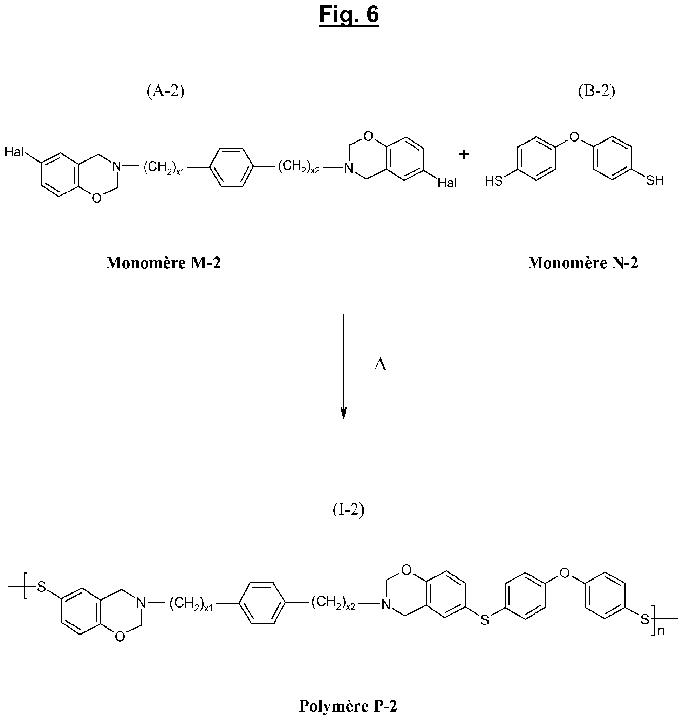

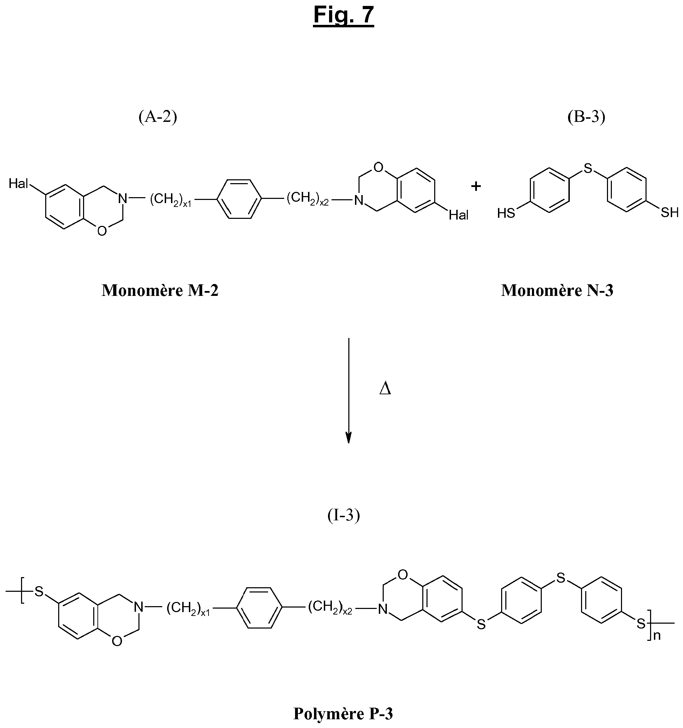

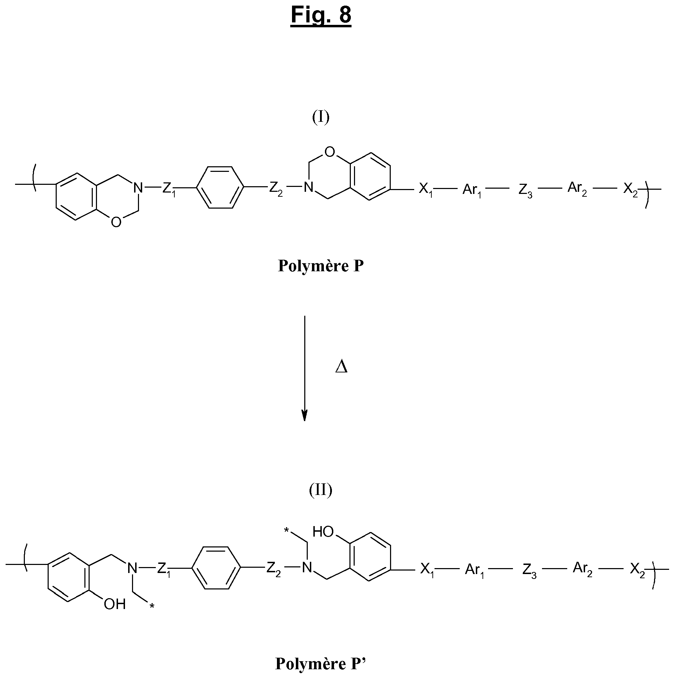

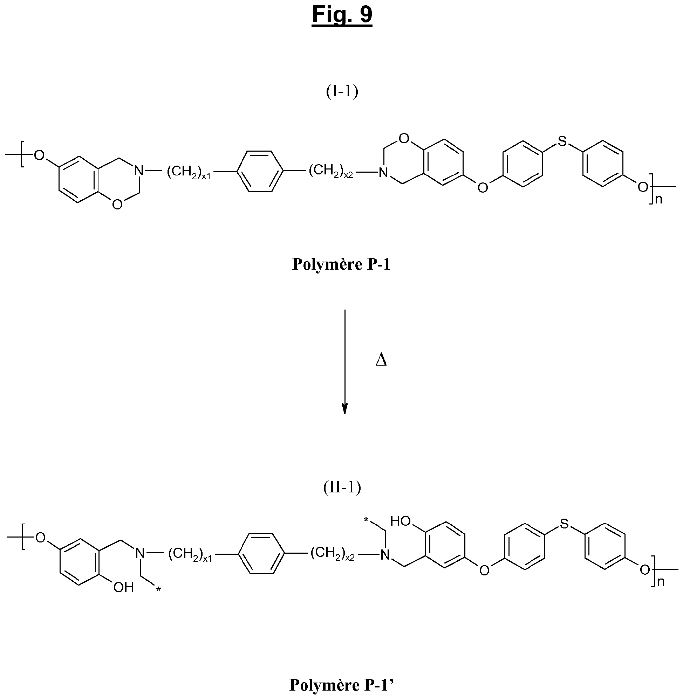

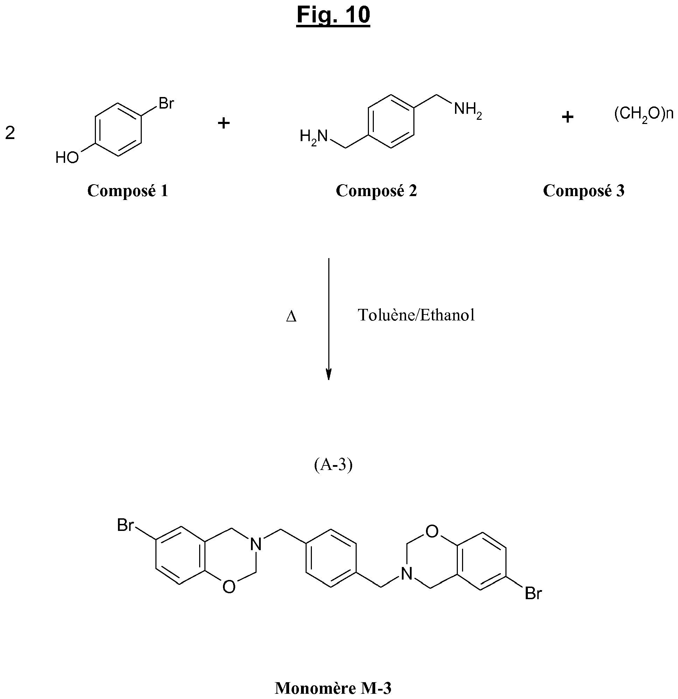

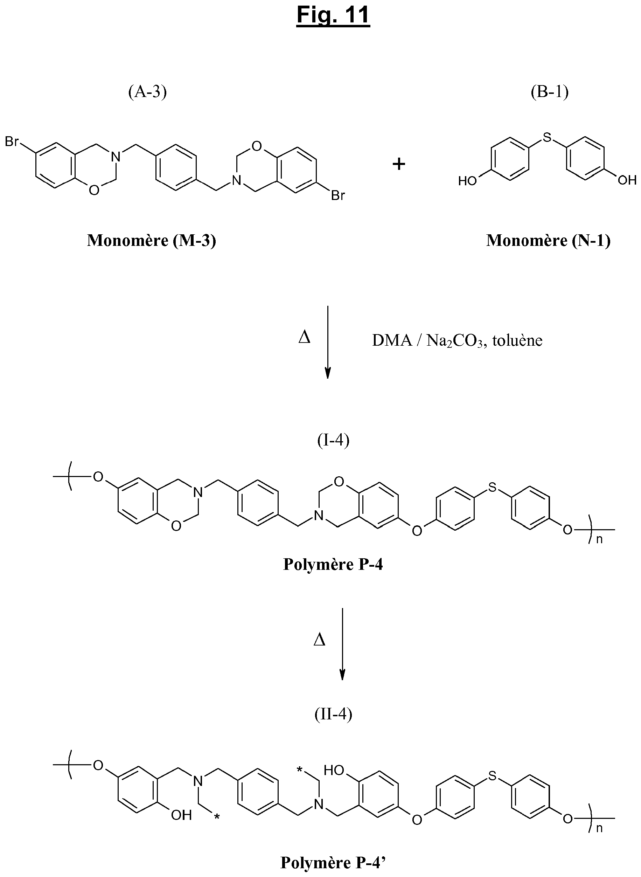

L'invention ainsi que ses avantages seront aisément compris à la lumière de la description détaillée et des exemples de réalisation qui suivent, ainsi que des figures 1 à 11 relatives à ces exemples qui représentent ou schématisent :
- le principe général de synthèse d'un composé benzoxazine à partir de trois composés, phénol, formaldéhyde et amine (R résidu de l'amine) (Fig. la) ;
- le mécanisme d'ouverture, par apport thermique, du cycle oxazine («*ring-opening*») d'un tel composé benzoxazine (Fig. 1b) ;
- un schéma de synthèse général, à partir d'un phénol halogéné (Hal représentant un halogène), de paraformaldéhyde et d'une diamine aromatique spécifique, d'une benzoxazine halogénée conforme à l'invention, de formule (A), utilisable comme monomère (Monomère noté « M ») pour la synthèse d'une polybenzoxazine (Fig. 2) ;
- un schéma de synthèse possible, à partir de phénol halogéné, p-formaldéhyde et d'une diamine spécifique du type aromatique, d'une benzoxazine halogénée particulière selon l'invention, de formule (A-1), utilisable comme monomère (Monomère noté M-1) pour la synthèse d'une polybenzoxazine particulière (Fig. 3) ;
- un schéma de synthèse général d'un polymère polybenzoxazine (Polymère noté « P ») à partir de la benzoxazine halogénée de l'invention de formule (A) (Monomère M) de la Fig. 2 et d'un autre monomère de formule générique (B) (Monomère noté « N ») du type diol ou thiol aromatique (Fig. 4) ;
- un schéma de synthèse d'un polymère polybenzoxazine particulier (Polymère noté P-1) à partir d'une benzoxazine halogénée particulière selon l'invention de formule (A-2) (Monomère M-2) et d'un autre monomère particulier de formule (B-1) (Monomère N-1) du type diol aromatique soufré (porteur d'une fonction thioéther) (Fig. 5) ;
- un schéma de synthèse d'une autre polybenzoxazine (Polymère noté P-2) à partir de la benzoxazine halogénée particulière selon l'invention de formule (A-2) (Monomère M-2) de la Fig. 5 précédente et d'un autre monomère particulier de formule (B-2) (Monomère N-2) du type thiol aromatique (porteur d'une fonction éther) (Fig. 6) ;
- un schéma de synthèse d'une autre polybenzoxazine (Polymère noté P-3) à partir de la benzoxazine halogénée selon l'invention de formule (A-2) (Monomère M-2) et d'un autre monomère particulier de formule (B-3) (Monomère N-3) du type thiol aromatique (porteur d'une fonction thioéther) (Fig. 7) ;
- la polybenzoxazine (Polymère noté ici P') de la Fig. 4 une fois ses cycles oxazine ouverts après traitement thermique du Polymère P (Fig. 8) ;
- la polybenzoxazine particulière (Polymère noté P-1') de la Fig. 5, une fois ses cycles oxazine ouverts après traitement thermique du Polymère P-1 (Fig. 9) ;
- le schéma de synthèse, à partir de phénol bromé (composé 1), p-formaldéhyde (composé 3) et d'une diamine spécifique aromatique (composé 2), d'une dibenzoxazine bromée particulière selon l'invention de formule (A-3) (Monomère noté M-3) utilisable pour la synthèse de polybenzoxazines (Polymère P-4 et P-4' de la figure 11) (Fig. 10) ;
- le schéma de synthèse d'une polybenzoxazine particulière (Polymère noté P-4) à partir de la benzoxazine halogénée particulière selon l'invention de formule (A-3) (Monomère M-3) de la Fig. 10 précédente et du monomère particulier de formule (B-1) (Monomère N-1), ainsi que la structure de ce polymère une fois ses cycles oxazine ouverts (Polymère noté P-4') (Fig. 11).

### 4. DESCRIPTION DETAILLEE DE L'INVENTION

On rappellera tout d'abord que les benzoxazines sont des composés de formule générale :

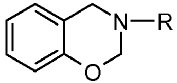

La figure la annexée rappelle le principe général de synthèse d'une benzoxazine, ici à partir (réaction de condensation) d'une molécule de phénol, de deux molécules de formaldéhyde et d'une amine (R désignant le résidu de l'amine), avec élimination de deux molécules d'eau.

La figure 1b rappelle quant à elle le mécanisme d'ouverture («*ring-opening*») du cycle oxazine d'un tel composé lors d'un apport thermique (représenté par le symbole Δ).

De nombreux composés ou monomères benzoxazines peuvent être ainsi synthétisés en utilisant divers phénols et amines selon leurs types de substituants. Ces groupes substituants peuvent fournir ensuite des sites polymérisables et permettre la synthèse de divers polymères benzoxazine (ou polybenzoxazines).

Benzoxazines et polybenzoxazines qui en sont issues sont des produits aujourd'hui bien connus de l'homme du métier ; pour ne citer que quelques exemples de publication, on peut mentionner les articles *« Polybenzoxazines* - *New high performance thermosetting resins : synthesis and properties »* ; N.N. Ghosh et al., Prog. Polym. Sci. 32 (2007), 1344-1391, ou « Recent Advancement on Polybenzoxazine - A newly Developed High Performance Thermoset », Y. Yaggi et al., J. Polym. Sci. Part A: Polym. Chem. : Vol. 47 (2009), 5565-5576, ainsi que par exemple les brevets ou demandes de brevet US 5 543 516, WO 2013/148408.

Comme expliqué en détail dans les documents ci-dessus, les polybenzoxazines ont la capacité remarquable, à haute température (par exemple, typiquement supérieure à 150°C voire supérieure à 200°C selon leur microstructure particulière), d'ouvrir leurs cycles oxazine et de conduire ainsi à des structures de résines polyphénoliques thermodurcissables.

La benzoxazine spécifique de l'invention, dénommée « Monomère M» dans la présente demande, est du type halogénée ; elle répond à la formule générique (A) qui suit, Hal représentant un (au moins un, c'est-à-dire un ou plusieurs) halogène :

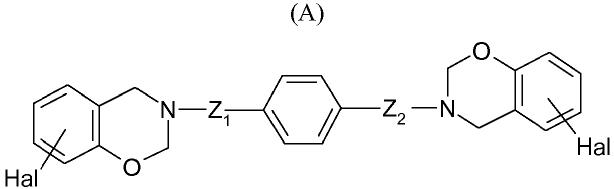

La figure 2 annexée en donne le schéma de synthèse général, sous apport thermique et avec élimination d'eau, à partir d'un phénol halogéné, de p-formaldéhyde et d'une diamine.

Dans la formule (A) ci-dessus, Z₁ et Z₂, identiques ou différents, représentent un groupement de liaison (espaceur *ou « spacer »*) au moins divalent c'est-à-dire qu'ils pourraient comporter plus de deux liaisons covalentes, par exemple trois ou quatre liaisons covalentes. De préférence, Z₁ et Z₂ sont divalents, c'est-à-dire ne comportent que deux liaisons covalentes.

Z₁ et Z₂ sont aliphatiques. Ces groupements, pouvant être éthyléniquement saturés ou insaturés, comportent de 1 à 12 atomes de carbone Selon un mode de réalisation particulier de l'invention, Z₁ et Z₂, identiques ou différents, représentent un groupement aliphatique comportant 1 à 12, plus préférentiellement 1 à 8, en particulier 1 à 6 atomes de carbone. Plus préférentiellement encore, Z₁ et Z₂, identiques ou différents, représentent un enchaînement (poly)alkylène (ou alkylidène) comportant 1 à 8, de préférence 1 à 6, en particulier 1 à 4 atomes de carbone.

Plus particulièrement, le composé de l'invention répond à la formule : 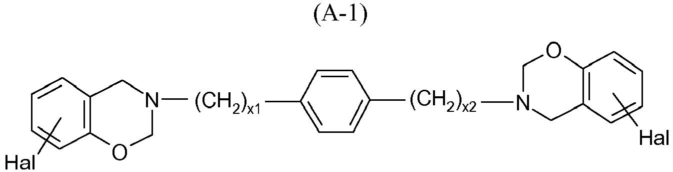 dans laquelle x₁ et x₂, identiques ou différents, représentent un entier de 1 à 8, de préférence de 1 à 6, en particulier de 1 à 4.

Chaque noyau benzénique des deux cycles oxazine du Monomère M est porteur d'au moins un (c'est-à-dire un ou plusieurs) halogène, cet halogène étant de préférence le brome (Br), le chlore (Cl) ou le fluor (F), plus préférentiellement le brome.

En outre, dans ce monomère de formule (A), un ou plusieurs atomes d'hydrogène d'au moins un ou de chacun de ces noyaux benzéniques peuvent être substitués (ou non) par différents substituants, par exemple par des groupes fonctionnels susceptibles de favoriser l'adhésion du polymère au métal et/ou au caoutchouc. De même, au moins un (c'est-à-dire un ou plusieurs) atome d'hydrogène du groupe phénylène (ou noyau benzénique central disposé entre Z₁ et Z₂) pourrait être également substitué par différents substituants, par exemple par de tels groupes fonctionnels susceptibles également de favoriser l'adhésion du polymère au métal et/ou au caoutchouc.

De préférence, chaque noyau benzénique des deux cycles oxazine du composé selon l'invention est porteur d'un seul halogène (Hal) ou au maximum de deux, plus préférentiellement d'un et d'un seul halogène, ce dernier étant plus préférentiellement situé en position para de l'oxygène du cycle oxazine.

Ainsi, selon un mode de réalisation particulièrement préférentiel, le composé de l'invention répond à la formule : 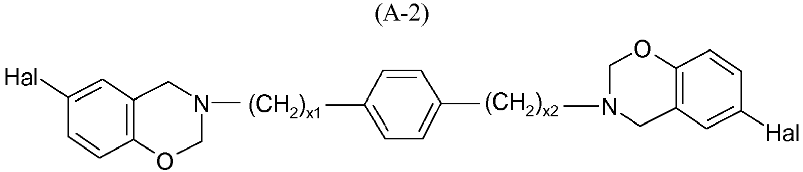 dans laquelle x₁ et x₂, identiques ou différents, représentent un entier de 1 à 8, de préférence de 1 à 6.

Selon un mode de réalisation particulièrement préférentiel, Hal représente le brome.

La figure 3 illustre un schéma de synthèse possible, à partir d'une diamine spécifique du type aromatique, d'une benzoxazine halogénée particulière selon l'invention, de formule (A-1), cette benzoxazine étant utilisable comme monomère (Monomère noté M-1) pour la synthèse ultérieure d'une polybenzoxazine. On note que Z₁ et Z₂ représentent respectivement -(CH₂)ₓ₁- et -(CH₂)ₓ₂-), formules dans lesquelles les symboles « x₁ » et « x₂ » représentent un nombre entier variant de préférence de 1 à 12, plus préférentiellement de 1 à 8, en particulier de 1 à 6. Une telle synthèse sera décrite plus en détail dans les exemples de réalisation qui suivent (Fig. 10).

La benzoxazine conforme à l'invention de formule (A) précédemment décrite est particulièrement destinée (à titre de Monomère M) à la synthèse d'une polybenzoxazine par polycondensation, en particulier par polycondensation avec au moins un composé diol ou thiol aromatique à titre de deuxième monomère (« Monomère N ») ayant pour formule (B) :

HX₁ - Ar₁ - Z₃ - Ar₂ - X₂H

dans laquelle :
- X₁ et X₂, identiques ou différents, représentent O (oxygène ; cas d'un monomère diol) ou S (soufre ; cas d'un monomère thiol) ;
- Ar₁ et Ar₂, identiques ou différents, représentent un groupe phénylène ;
- Z₃ représente O ou (S)ₙ, le symbole « n » représentant un nombre entier égal à 1 (cas d'un seul atome de soufre) ou supérieur à 1 (cas de plusieurs atomes de soufre).

Dans la formule générique (B) ci-dessus, on a préférentiellement au moins une des caractéristiques suivantes qui est vérifiée :
- X₁ et X₂ représentent chacun soit un atome de soufre, soit un atome d'oxygène ;
- Z₃ représente O ou S (soit « n » égal à 1), plus préférentiellement S.

Plus préférentiellement, c'est l'ensemble des caractéristiques préférentielles ci-dessus qui est vérifié simultanément.

En outre, dans la formule (B) ci-dessus, un ou plusieurs atomes d'hydrogène d'au moins un ou de chaque groupe Ar₁ et Ar₂ peuvent être substitués (ou non) par un seul ou plusieurs substituants, identiques ou différents, par exemple par des groupes fonctionnels susceptibles de favoriser l'adhésion du polymère au métal et/ou au caoutchouc.

A titre d'exemples de Monomères N (diols ou thiols) convenant préférentiellement à l'invention peuvent être cités les Monomères respectivement notés N-1, N-2 et N-3 sur les figures 5, 6 et 7 commentées ultérieurement, monomères de formules respectives (B-1), (B-2) et (B-3) ci-dessous :

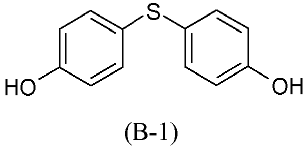

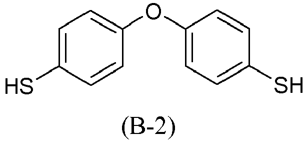

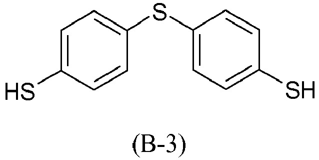

Ainsi, la benzoxazine conforme à l'invention de formule (A) est particulièrement destinée à la synthèse d'une polybenzoxazine (Polymère noté «P») comportant des unités structurelles récurrentes comportent au moins un motif répondant aux formule (I) (avant ouverture des cycles oxazine) ou formule (II) (après ouverture des cycles) ci-dessous : 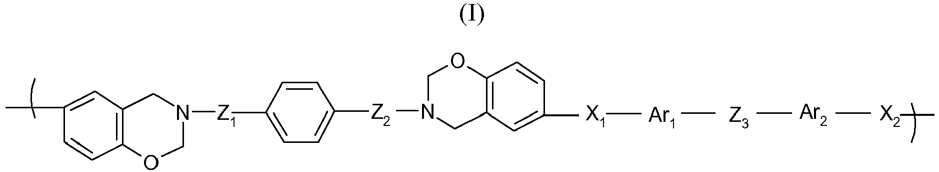 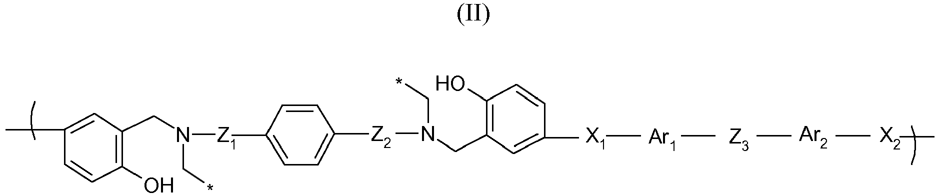 formules (I) et (II) ci-dessus dans lesquelles Z₁, Z₂ et Z₃ ont la définition principale et les définitions préférentielles déjà données précédemment.

Par polymère doit être entendu ici tout homopolymère ou copolymère, notamment copolymère à blocs, avec des unités structurelles récurrentes comportent au moins un motif de formule (I) ou (II) ci-dessus ; ce polymère peut bien entendu comporter à la fois des motifs de formule (I) et des motifs de formule (II).

Dans la formule (II) ci-dessus, l'homme du métier comprendra immédiatement que les deux symboles «^{∗}» (identiques ou différents) représentent un rattachement quelconque du motif à un atome de carbone ou à un hétéroatome (choisi de préférence parmi O, S, N et P), rattachement ou liaison résultant de l'ouverture des cycles oxazine.

La figure 4 représente un schéma général de synthèse, par polycondensation, d'une telle polybenzoxazine (Polymère P) à partir de la benzoxazine halogénée selon l'invention de formule (A) de la Fig. 2 (Monomère M) et d'un autre monomère, de formule générique (B), qui est du type diol ou thiol aromatique (Monomère N).

La polybenzoxazine de la figure 4, plus exactement au moins une partie de ses unités récurrentes, a été également représentée à la figure 8, avant (Polymère P) et après (Polymère P') ouverture des cycles oxazine.

La figure 5 représente un schéma de synthèse particulier d'une polybenzoxazine spécifique (Polymère noté P-1) de formule (I-1), à partir d'une benzoxazine halogénée particulière selon l'invention (Monomère M-2) de formule (A-2) et d'un autre monomère spécifique (Monomère N-1) de formule (B-1) du type diol aromatique soufré (4,4'-thiodiphenol).

Dans cet exemple, on note en particulier que, selon un mode de réalisation préférentiel de l'invention déjà décrit, chaque noyau benzénique de la benzoxazine (Monomère M-2) conforme à l'invention est porteur d'un et un seul halogène (Hal), plus préférentiellement du brome, cet halogène étant plus particulièrement situé en position para de l'oxygène du cycle oxazine.

Cette polybenzoxazine de la figure 5, ou plus exactement au moins une partie de ses unités récurrentes, a également été représentée à la figure 9, avant (Polymère P-1) et après (P-1') ouverture de ses cycles oxazine suite à un apport thermique suffisant.

Ainsi, selon un mode de réalisation particulièrement préférentiel, la polybenzoxazine issue de la benzoxazine de l'invention se caractérise par des unités récurrentes comportant au moins un motif répondant aux formules particulières (I-1) (avant ouverture des cycles benzoxazine) ou (II-1) (après ouverture des cycles) : 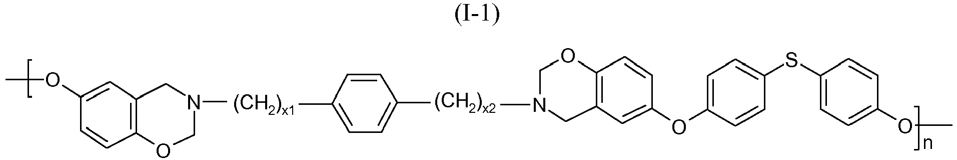 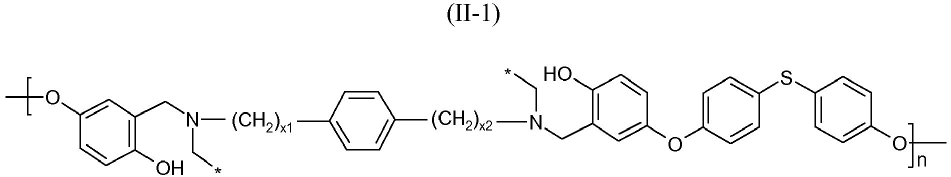 La figure 6 représente un autre schéma de synthèse particulière d'une autre polybenzoxazine spécifique (Polymère noté P-2), de formule (I-2), à partir de la benzoxazine halogénée spécifique de l'invention (Monomère M-2) précédente et d'un autre monomère spécifique (Monomère N-2) de formule (B-2), du type thiol aromatique (porteur en outre d'une fonction éther).

La figure 7 représente un autre schéma de synthèse particulière d'une autre polybenzoxazine spécifique (Polymère noté P-3), de formule (I-3), à partir de la benzoxazine halogénée spécifique de l'invention (Monomère M-2) précédente et d'un autre monomère spécifique (Monomère N-3) du type thiol aromatique (porteur en outre d'une fonction thioéther).

Dans ces exemples des figures 6 et 7, comme pour la figure 5 précédente, on note en particulier que, selon un mode de réalisation préférentiel de l'invention déjà indiqué, chaque noyau benzénique des deux cycles oxazine de la benzoxazine de l'invention est porteur d'un et un seul halogène (Hal), plus préférentiellement du brome, situé plus particulièrement en position para de l'oxygène du cycle oxazine.

Comme déjà indiqué, les figures 8 et 9 représentent également les polybenzoxazines respectivement notées P' et P-1' des Fig. 4 et Fig. 5, une fois leurs cycles oxazine ouverts.

Typiquement, la polybenzoxazine issue du composé benzoxazine de l'invention peut comporter de dix à plusieurs centaines, préférentiellement de 50 à 300 unités structurelles à motifs de formule (I) et/ou (II), en particulier d'unités structurelles telles que représentées à titre d'exemples aux figures 5 à 9 et 11.

Cette polybenzoxazine issue de la benzoxazine de l'invention est avantageusement utilisable, à titre de primaire d'adhésion ou comme couche adhésive unique, pour revêtir un substrat en métal, tout au moins un substrat dont au moins la surface est au moins en partie métallique, et faire adhérer ce dernier à du caoutchouc. Elle est tout particulièrement utilisable sur tout type de renfort métallique, tel que par exemple un fil, un film ou un câble en acier, notamment en acier au carbone, destiné en particulier à renforcer une matrice de caoutchouc insaturé tel que du caoutchouc naturel. Pour faire adhérer le caoutchouc à la couche de polybenzoxazine, on pourra aussi utiliser tout système adhésif connu, par exemple une colle textile conventionnelle du type "RFL" (résorcinol-formaldéhyde-latex). L'homme du métier comprendra aisément que la connexion entre le substrat métallique pourvu de sa couche de polybenzoxazine et la couche de caoutchouc avec laquelle il est au contact, sera assurée définitivement lors de la cuisson (réticulation) finale de l'article en caoutchouc concerné.

### 5. EXEMPLES DE REALISATION DE L'INVENTION

Dans la présente demande, sauf indication expresse différente, tous les pourcentages (%) indiqués sont des % en masse.

Les essais qui suivent décrivent tout d'abord la synthèse d'un composé benzoxazine préférentiel (Monomère M-3) conforme à l'invention, de formule :

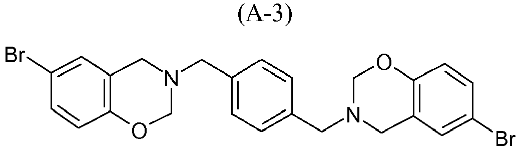

Puis on décrit la synthèse, à partir de ce monomère M-3, d'une polybenzoxazine (Polymère P-4) de formule (I-4) :

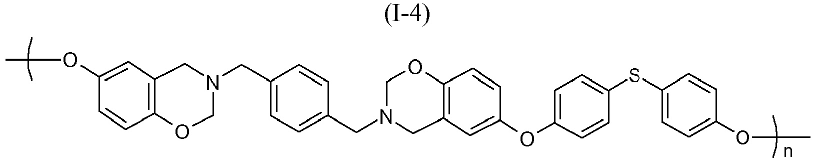

Enfin, des tests d'adhésion sont conduits pour illustrer l'excellente performance adhésive des polybenzoxazines issues des benzoxazines de l'invention.

### 5.1. Synthèse d'une benzoxazine halogénée conforme à l'invention (Monomère M-3)

On dispose pour cette synthèse d'un ballon rond à trois cols de 250 ml, équipé d'un thermomètre, d'une entrée d'azote, d'un agitateur magnétique et d'un réfrigérant.

La synthèse est réalisée selon le mode opératoire schématisé à la figure 10, comme expliqué en détail ci-après, à partir de trois composés : phénol halogéné (composé 1 ; 4-bromophénol ; produit Aldrich B75808), une diamine aromatique (composé 2 ; p-xylylène diamine ; produit TCI Europe D1018) et paraformaldéhyde (composé 3 ; produit Aldrich 158127), en présence de deux solvants (toluène et éthanol anhydres).

On verse dans le ballon le composé 1 (2 eq, 10,38 g, soit 60 mmol) puis de l'éthanol (51 ml). La présence d'éthanol est ici importante, empêchant la formation de produit intermédiaire du type triazine, instable. Sous agitation, on introduit ensuite le composé 2 (1 eq, 4,13 g soit 30 mmol), le composé 3 (4 eq, 3,60 g soit 120 mmol) et enfin le toluène (102 ml). Le milieu réactionnel est chauffé (environ 75°C) à reflux pendant 51 h, puis les solvants et résidus volatils sont distillés à 110°C (sous vide de 1 mbar) pour évaporation. Le produit final est ensuite lavé (100 ml méthanol) et séché ; on obtient ainsi une poudre de couleur jaune.

Cette poudre est placée dans du méthanol (100 ml pour 15 g de poudre) et on chauffe à reflux (65°C) pendant 30 min. Puis on laisse la solution refroidir à température ambiante (environ 20°C) pour cristallisation du monomère. Le produit solide obtenu est isolé par filtration (filtre Büchner). On obtient ainsi une poudre de couleur blanche, après un séchage à l'étuve sous vide à 50°C, durant toute une nuit (rendement de réaction égal à environ 82%).

Le spectre RMN ¹H (500 MHz) du Monomère M-3 ainsi synthétisé, dissous dans du THF-*d*8, a confirmé sa structure chimique, avec les résultats suivants:
*3.87 (s, 4H), 3.92 (s, 4H), 4.86 (s, 4H), 6.66-6.68 (d, 2H), 7.09-7.10 (d, 2H), 7.19-7.22 (d, 2H), 7.31 (s, 4H).*

### 5.2. Synthèse d'une polybenzoxazine (Polymère P-4)

Cette synthèse est réalisée selon le mode opératoire schématisé à la figure 11, comme décrit en détail ci-après, à partir de deux monomères : la benzoxazine de l'invention obtenue à l'étape précédente (Monomère M-3) et le diol aromatique soufré de formule (B-1) (4,4'-thiodiphenol ; Monomère N-1) déjà décrit à la figure 5 ; ceci en présence de carbonate de sodium (Na₂CO₃ ; produit Sigma Aldrich 13418), de solvants (anhydres) DMA (N,N-diméthylacétamide ; produit Sigma Aldrich 38839) et toluène (produit Acros Organics N°364411000). Les deux monomères (M-3 et N-1) sont préalablement séchés sous vide (10 mbar) à 60°C toute la nuit, de même pour le carbonate de sodium mais à une température de 150°C.

La synthèse est conduite dans un ballon rond à quatre cols de 100 ml, équipé d'une entrée d'azote, d'un thermomètre, d'un agitateur magnétique et d'un séparateur «Dean Stark » surmonté d'un réfrigérant et d'un pont de distillation (pourvu d'une calotte chauffante). L'appareillage est séché sous vide en utilisant un pistolet à air chaud jusqu'à ce que le thermomètre atteigne une température d'au moins 100°C dans le ballon de réaction. On laisse l'ensemble refroidir à température ambiante (20°C), puis l'appareillage est mis sous courant d'azote pendant toute la synthèse.

On introduit alors dans le ballon tout d'abord le Monomère M-3 (1 eq; 1,48 g soit 2,79 mmol) de formule (A-3), puis le Monomère N-1 de formule (B-1) (1 eq; 0,61 g soit 2,79 mmol). On ajoute ensuite 20 ml de DMA (solvant des deux monomères), puis à titre de base Na₂CO₃ (3 eq, 0,89 g soit 8,36 mol) en suspension dans 4 ml de toluène. Le tout est purgé sous N₂ pendant 5 min, puis on chauffe le milieu réactionnel à 105°C. Une fois cette température atteinte (température de calotte chauffante d'environ 115°C), le pont de distillation de l'appareillage Dean Stark est chauffé à 110°C (avec la calotte chauffante) afin de faciliter la distillation azéotropique (distillation eau/toluène) conduite pendant environ 90 min. Puis on augmente progressivement la température du milieu réactionnel, par pallier de 10°C toutes les 30 min, jusqu'à atteindre 130°C. On laisse à cette température durant 17 h, puis on laisse refroidir à température ambiante (20°C). Le mélange réactionnel est ensuite distillé à 90°C (vide 3 mbar) pour élimination des solvants et résidus volatils, puis le précipité solide ainsi obtenu est lavé avec 250 ml d'eau distillée ; au cours de ce lavage, pour extraire le carbonate, on ajoute de l'acide (HCl 1 % aqueux) goutte à goutte jusqu'à pH neutre. Le précipité est une nouvelle fois lavé avec 100 ml d'eau distillée, séché sous vide à 80°C pendant toute une nuit (environ 12 h).

Le Polymère P-4 de la Fig. 11 a été ainsi obtenu, comme attesté par l'analyse RMN ¹H (500 MHz) dans le solvant THF-*d*8, qui a donné les résultats suivants :
*7.35 (br, 4H), 7.21 (br, 2H), 7.08-7.14 (br, 6H), 6.62-6.68 (br, 6H), 4.87 (br, 4H), 3.1-4.4 (br, 8H). 3.1-4.4 (br, 8H).*

Ce Polymère P-4, sous forme d'une poudre de couleur rouge (rouille), a été également analysé par DSC (*Differential Scanning Calorimetry*) entre -80°C et +250°C selon une rampe de 10°C/min (appareil DSC "822-2" de Mettler Toledo ; atmosphère azote). L'analyse a montré au premier passage (entre -80°C et +250°C) une exothermie (correspondant à l'ouverture des cycles oxazine, et à la réticulation du polymère) au-delà de 200°C, avec un maximum à environ 230°C. Au cours des deuxième et troisième passages de DSC conduits entre -80°C et +250°C, aucune transition vitreuse apparente (Tg) n'a été visible.

### 5.3. Test d'adhésion dans un composite métal / caoutchouc

Une partie (650 mg) du Polymère P-4 précédemment préparé a été dissoute dans 8 ml de DMA (N,N-diméthylacétamide ; produit Sigma Aldrich 38839), ceci pour former une solution dont une fraction (0,7 ml) a été ensuite déposée de manière uniforme sur un ruban (film) en laiton de dimensions 10 cm x 2,5 cm et d'épaisseur 0,4 mm ; l'ensemble a été placé à l'étuve à 175°C (ventilation à l'air) durant 5 min, puis 5 min supplémentaires à 230°C sous vide afin d'une part d'éliminer toute trace de solvant et d'autre part d'ouvrir au moins en partie (c'est-à-dire totalement ou partiellement) les cycles oxazine du polymère, cette dernière étape s'accompagnant d'un changement prononcé de couleur du polymère, passant au orange foncé.

Après refroidissement à température ambiante, le ruban pourvu en surface de sa fine couche (épaisseur 5 à 10 µm) de polybenzoxazine ainsi formée, a été ensuite soumis à une opération d'encollage conventionnelle en deux temps (encollage deux bains), tout d'abord par immersion dans un premier bain aqueux (environ 94% d'eau) à base de résine époxy (polyglycérol polyglycidyl éther, environ 1%) et de composé isocyanate (bloqué caprolactame, environ 5%), première étape d'encollage suivie d'un séchage (2 min à 100°C) puis d'un traitement thermique (5 min à 200°C). Puis le ruban ainsi traité a été immergé dans un second bain aqueux de colle RFL (environ 81% en poids d'eau) à base de résorcinol (environ 2%), formol (environ 1%) et d'un latex de caoutchouc (environ 16% de caoutchoucs NR, SBR et VP-SBR) ; il a été enfin séché au four pendant 2 min à 130°C, puis traité thermiquement 5 min à 200°C.

Le ruban en laiton ainsi revêtu du film de polybenzoxazine puis encollé, a été ensuite placé entre deux couches de composition de caoutchouc conventionnelle pour armature de ceinture de bandage pour véhicule tourisme, composition à base de caoutchouc naturel, de noir de carbone et silice à titre de charge, et d'un système de vulcanisation (soufre et accélérateur sulfénamide); cette composition étant dépourvue de sel de cobalt. Puis l'éprouvette de composite métal / caoutchouc ainsi préparée a été placée sous presse et le tout cuit (vulcanisé) à 150°C pendant 30 min sous une pression de 20 bar.

Après vulcanisation du caoutchouc, on a obtenu un excellent collage entre la matrice de caoutchouc et le ruban métallique malgré l'absence de sel de cobalt dans la matrice de caoutchouc : lors de tests de pelage (à 20°C), on a constaté en effet que la rupture se produisait systématiquement dans la matrice de caoutchouc elle-même et non à l'interphase entre métal et caoutchouc. D'autres essais de collage ont été conduits sur un ruban en acier clair (non revêtu) ; ils ont révélé eux aussi une excellente adhésion au caoutchouc (rupture systématique dans la matrice de caoutchouc).

En conclusion, la benzoxazine selon l'invention permet la synthèse de polymères offrant aux renforts métalliques l'avantage majeur de pouvoir ensuite être collés à des matrices de caoutchouc en utilisant de simples colles textiles telles que des colles RFL, ou encore directement (c'est-à-dire sans emploi de telles colles) à ces matrices de caoutchouc, par exemple lorsque ces dernières contiennent des élastomères insaturés fonctionnalisés appropriés tels que des élastomères époxydés. Ainsi, peuvent être utilisés des renforts métalliques revêtus ou non de couches métalliques adhésives telles que du laiton, ainsi que des matrices de caoutchouc environnantes dépourvues de sels métalliques, en particulier de sels de cobalt.

En outre, ceci constituant un avantage notable comparativement aux autres polymères connus décrits en introduction du présent mémoire, les polybenzoxazines issues des benzoxazines de l'invention ont la capacité remarquable, à haute température, d'ouvrir leurs cycles oxazine et de conduire ainsi à une structure de résine polyphénolique thermodurcissable. Ceci leur confère une meilleure stabilité thermique. Leur microstructure spécifique permet enfin, très avantageusement, d'ajuster la flexibilité de la molécule selon les applications particulières visées.

## Revendications

1. Composé benzoxazine halogénée répondant à la formule (le symbole Hal représentant au moins un halogène) : 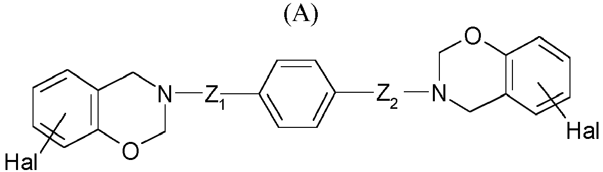 dans laquelle :
- chaque noyau benzénique des deux cycles oxazine est porteur d'au moins un halogène ;
- Z₁ et Z₂, identiques ou différents, représentent un groupement de liaison au moins divalent, aliphatique comportant de 1 à 12 atomes de carbone.

2. Composé selon la revendication 1, dans lequel Z₁ et Z₂, identiques ou différents, représentent un groupement aliphatique comportant de 1 à 8 atomes de carbone.

3. Composé selon la revendication 2, dans lequel Z₁ et Z₂, identiques ou différents, représentent un enchaînement (poly)alkylène comportant 1 à 8, de préférence 1 à 6 atomes de carbone.

4. Composé selon la revendication 3, répondant à la formule : 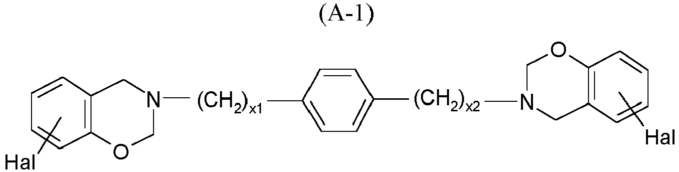 dans laquelle x₁ et x₂, identiques ou différents, représentent un entier de 1 à 8, de préférence de 1 à 6.

5. Composé selon l'une quelconque des revendications 1 à 4, dans lequel Hal représente le brome, le chlore ou le fluor, de préférence Hal représente le brome.

6. Composé selon l'une quelconque des revendications 1 à 5, dans lequel chaque noyau benzénique des deux cycles oxazine est porteur d'un seul halogène.

7. Composé selon la revendication 6, dans lequel l'halogène porté par chaque noyau benzénique des deux cycles oxazine est situé en position para de l'oxygène du cycle oxazine.

8. Composé selon l'une quelconque des revendications 4 à 7, répondant à la formule : 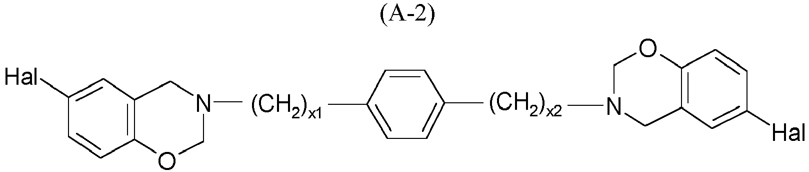 dans laquelle x₁ et x₂, identiques ou différents, représentent un entier de 1 à 8, de préférence de 1 à 6.

9. Composé selon la revendication 8, répondant à la formule :

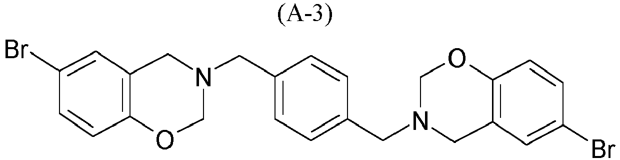

10. Utilisation d'un composé selon l'une quelconque des revendications 1 à 9, pour la synthèse d'une polybenzoxazine.

11. Polybenzoxazine obtenue par polycondensation d'au moins un composé selon l'une quelconque des revendications 1 à 9.

12. Procédé de synthèse d'une polybenzoxazine, par polycondensation d'un composé selon l'une quelconque des revendications 1 à 9.

13. Procédé selon la revendication 12, par polycondensation, à titre de premier monomère, d'un composé selon l'une quelconque des revendications 1 à 9, avec au moins, à titre de deuxième monomère, un composé diol ou thiol aromatique.

14. Procédé selon la revendication 13, le composé diol ou thiol aromatique répondant à la formule (B):
HX₁ - Ar₁ - Z₃ - Ar₂ - X₂H
dans laquelle
- X₁ et X₂, identiques ou différents, représentent O ou S ;
- Ar₁ et Ar₂, identiques ou différents, représentent un groupe phénylène ;
- Z₃ représente O ou (S)ₙ, le symbole «n» représentant un nombre entier égal ou supérieur à 1.

15. Procédé selon la revendication 14, le composé diol ou thiol aromatique répondant à au moins une des formules (B-1), (B-2 ou (B-3) ci-dessous :

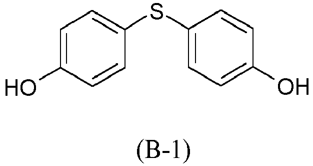

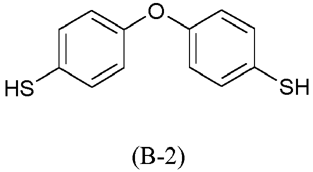

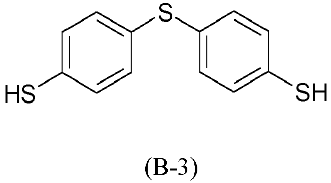

## Patentansprüche

1. Halogenierte Benzoxazinverbindung der Formel (wobei das Symbol Hal für mindestens ein Halogen steht): 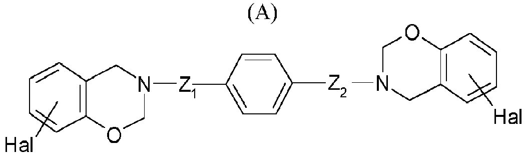 in der:
- jeder Benzolkern der beiden Oxazinringe mindestens ein Halogen trägt;
- Z₁ und Z₂ gleich oder verschieden sind und für eine mindestens zweiwertige aliphatische Verknüpfungsgruppe mit 1 bis 12 Kohlenstoffatomen stehen.

2. Verbindung nach Anspruch 1, wobei Z₁ und Z₂ gleich oder verschieden sind und für eine aliphatische Verknüpfungsgruppe mit 1 bis 8 Kohlenstoffatomen stehen.

3. Verbindung nach Anspruch 2, wobei Z₁ und Z₂ gleich oder verschieden sind und für eine (Poly)alkylen-sequenz mit 1 bis 8 und vorzugsweise 1 bis 6 Kohlenstoffatomen stehen.

4. Verbindung nach Anspruch 3 der Formel: 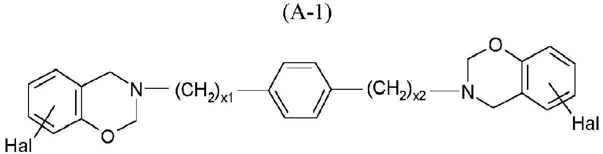 in der x₁ und x₂ gleich oder verschieden sind und für eine ganze Zahl von 1 bis 8 und vorzugsweise von 1 bis 6 stehen.

5. Verbindung nach einem der Ansprüche 1 bis 4, wobei Hal für Brom, Chlor oder Fluor steht und vorzugsweise Hal für Brom steht.

6. Verbindung nach einem der Ansprüche 1 bis 5, wobei jeder Benzolkern der beiden Oxazinringe ein einziges Halogen trägt.

7. Verbindung nach Anspruch 6, wobei das Halogen, das jeder Benzolkern der beiden Oxazinringe trägt, in para-Position zum Sauerstoff des Oxazinrings steht.

8. Verbindung nach einem der Ansprüche 4 bis 7 der Formel: 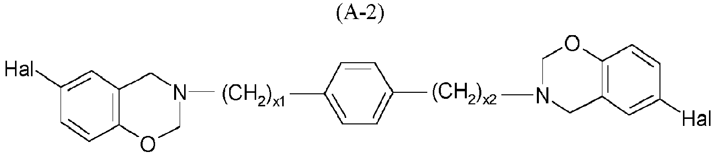 in der x₁ und x₂ gleich oder verschieden sind und für eine ganze Zahl von 1 bis 8 und vorzugsweise von 1 bis 6 stehen.

9. Verbindung nach Anspruch 8 der Formel:

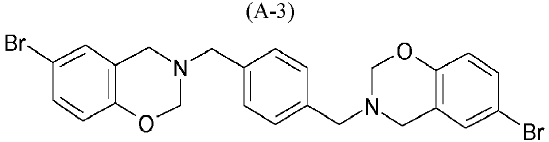

10. Verwendung einer Verbindung nach einem der Ansprüche 1 bis 9 zur Synthese eines Polybenzoxazins.

11. Polybenzoxazin, erhalten durch Polykondensation mindestens eine Verbindung nach einem der Ansprüche 1 bis 9.

12. Verfahren zur Synthese eines Polybenzoxazins durch Polykondensation einer Verbindung nach einem der Ansprüche 1 bis 9.

13. Verfahren nach Anspruch 12 durch Polykondensation einer Verbindung nach einem der Ansprüche 1 bis 9 als erstes Monomer mit mindestens einer aromatischen Diol- oder Thiolverbindung als zweites Monomer.

14. Verfahren nach Anspruch 13, wobei die aromatische Diol- oder Thiolverbindung der Formel (B) entspricht:
HX₁- Ar₁ - Z₃ - Ar₂ - X₂H
in der
- X₁ und X₂ gleich oder verschieden sind und für O oder S stehen;
- Ar₁ und Ar₂ gleich oder verschieden sind und für eine Phenylengruppe stehen;
- Z₃ für O oder (S)ₙ steht, wobei das Symbol "n" für eine ganze Zahl gleich oder größer als 1 steht.

15. Verfahren nach Anspruch 14, wobei die aromatische Diol- oder Thiolverbindung mindestens einer der nachstehenden Formeln (B-1), (B-2) oder (B-3) entspricht:

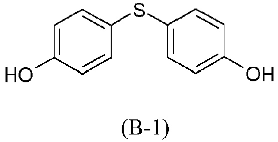

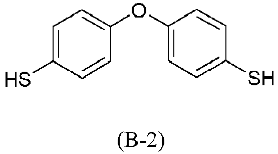

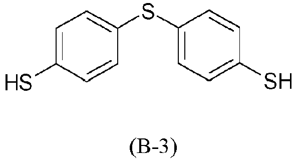

## Claims

1. Halogenated benzoxazine compound corresponding to the formula (the symbol Hal representing at least one halogen): 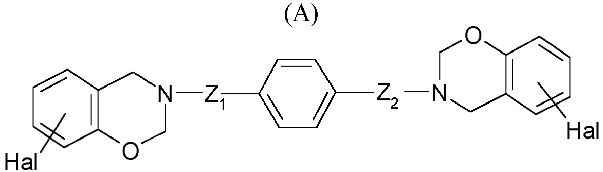 in which:
- each benzene nucleus of the two oxazine rings bears at least one halogen;
- Z₁ and Z₂, which are identical or different, represent an aliphatic, at least divalent, bonding group, comprising from 1 to 12 carbon atoms.

2. Compound according to Claim 1, in which Z₁ and Z₂, which are identical or different, represent an aliphatic group comprising from 1 to 8, carbon atoms.

3. Compound according to Claim 2, in which Z₁ and Z₂, which are identical or different, represent a (poly)alkylene sequence comprising from 1 to 8, preferably from 1 to 6, carbon atoms.

4. Compound according to Claim 3, corresponding to the formula: 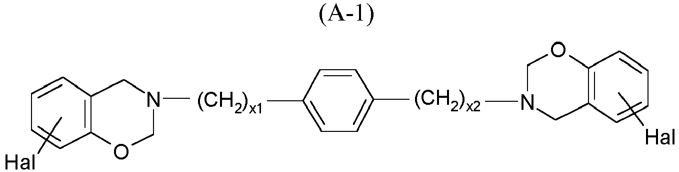 in which x₁ and x₂, which are identical or different, represent an integer from 1 to 8, preferably from 1 to 6.

5. Compound according to any one of Claims 1 to 4 in which Hal represents bromine, chlorine or fluorine, preferably Hal represents bromine.

6. Compound according to any one of Claims 1 to 5, in which each benzene nucleus of the two oxazine rings bears a single halogen.

7. Compound according to Claim 6, in which the halogen borne by each benzene nucleus of the two oxazine rings is located in the para position with respect to the oxygen of the oxazine ring.

8. Compound according to any one of Claims 4 to 7, corresponding to the formula: 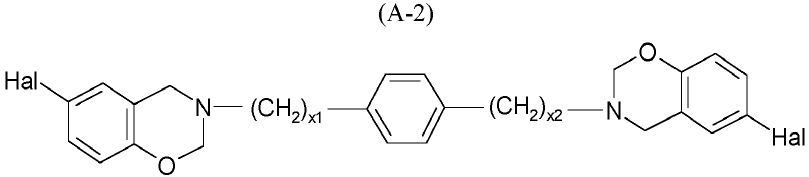 in which x₁ and x₂, which are identical or different, represent an integer from 1 to 8, preferably from 1 to 6.

9. Compound according to Claim 8, corresponding to the formula:

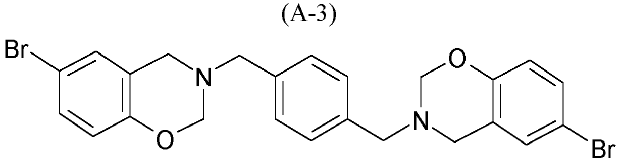

10. Use of a compound according to any one of Claims 1 to 9 in the synthesis of a polybenzoxazine.

11. Polybenzoxazine resulting from at least one compound according to any one of Claims 1 to 9.

12. Process for the synthesis of a polybenzoxazine by polycondensation of a compound according to any one of Claims 1 to 9.

13. Process according to Claim 12, by polycondensation, as first monomer, of a compound according to any one of Claims 1 to 9 with at least, as second monomer, an aromatic diol or thiol compound.

14. Process according to Claim 13, the aromatic diol or thiol compound corresponding to the formula (B):
HX₁- Ar₁ - Z₃ - Ar₂ - X₂H
in which:
- X₁ and X₂, which are identical or different, represent O or S;
- Ar₁ and Ar₂, which are identical or different, represent a phenylene group;
- Z represents O or (S)ₙ, the symbol "n" representing an integer equal to or greater than 1.

15. Process according to Claim 14, the aromatic diol or thiol compound corresponding to at least one of the formulae (B-1), (B-2) or (B-3) below:
